# EUROPEAN PATENT APPLICATION

(11) **EP 2 508 173 A1**
(43) Date of publication of application: **10.10.2012**
(21) Application number: 11161657.9
(22) Date of filing: 08.04.2011
(51) Int. Cl.: A61K 9/16, A61K 9/20, A61K 31/222

(54) **Stabilized pharmaceutical composition comprising fesoterodine**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The present invention relates to a granulate and a pharmaceutical composition comprising fesoterodine or a salt or a solvate thereof and stabilizer, in particular to a pharmaceutical composition comprising fesoterodine or a salt or a solvate thereof and sucrose, polyethylene glycol, cyclodextrin, maltodextrin and combinations thereof and to a process for its preparation. The granulate and the pharmaceutical composition are particularly useful as a medicament, especially for the treatment of urinary incontinence. The present invention relates to use of sucrose, polyethylene glycol, cyclodextrin, maltodextrin and combinations thereof for stabilizing fesoterodine or a salt or a solvate thereof in a pharmaceutical composition.

## Description

### Field of the Invention

The present invention relates to a granulate comprising fesoterodine, or a salt or a solvate thereof, and a new stabilizer, and to a pharmaceutical composition comprising said granulate. The present invention relates to a process for the preparation of pharmaceutical composition comprising fesoterodine, or a salt or a solvate thereof, and stabilizer. The pharmaceutical composition is particularly useful as a medicament, especially for the treatment of urinary incontinence. The present invention relates to use of sucrose, polyethylene glycol, cyclodextrin, maltodextrin or combinations thereof for stabilizing fesoterodine, or a salt or a solvate thereof, in a pharmaceutical composition.

### Description of Background Art

Fesoterodine ([2-[(1R)-3-(Di(propan-2-yl)amino)-1-phenylpropyl]-4-(hydroxymethyl)phenyl] 2-methylpropanoate) is a muscarinic receptor antagonist used for the treatment of overactive bladder including urinary incontinence. Fesoterodine substance was disclosed in WO 98/43942 and WO 99/58478, while salts of fesoterodine were described in EP 1230209.

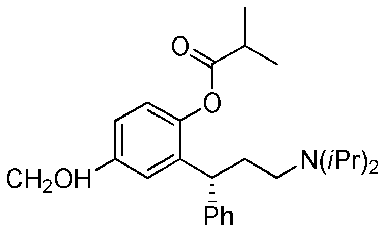

Fesoterodine is a tolterodine (3-(2-hydroxy-5-methylphenyl)-*N,N*-diisopropyl-3-phenylpropylamine) prodrug that is converted to an active molecule hydroxy metabolite 2-(3-(diisopropylamino)-1-phenylpropyl)-4-(hydroxymethyl)phenol (hydroxytolterodine) in the body. However, fesoterodine substance is prone to conversion to hydroxytolterodine under humid environment and at increased temperature. Said degradation of fesoterodine to hydroxytolterodine in the pharmaceutical formulation is undesirable. Therefore, there is a need to provide a pharmaceutical composition comprising fesoterodine that is stable against fesoterodine degradation over an extended period of time.

So far, two solutions regarding fesoterodine stability in the pharmaceutical composition have been presented. In WO 2007/141298 pharmaceutical excipients such as xylitol, sorbitol, polydextrose, isomalt and combinations thereof were found to be able to significantly slow down the degradation of fesoterodine under stress conditions. WO 2010/043408 discloses a microencapsuled fesoterodine composition which, distinct from a homogenous mixture of fesoterodine-particle with a matrix, is composed of a particle containing fesoterodine and a shell surrounding the fesoterodine-containing particle. However, the prior art proposed compositions require exotic stabilizers not commonly used in the pharmaceutical industry (cf. WO 2007/141298), or complex-structured pharmaceutical composition (cf. WO 2010/043408).

Therefore, there is an unmet need for new pharmaceutical composition comprising fesoterodine that are stable against fesoterodine degradation over an extended period of time.

### Summary of the invention

The aspects, advantageous features and preferred embodiments of the present invention summarized in the following items, respectively alone or in combination, further contribute to solving the object of the invention:
1. A granulate comprising fesoterodine or a salt or a solvate thereof, and a stabilizer selected from a group consisting of sucrose, polyethylene glycol, cyclodextrin, maltodextrin and combinations thereof.
2. The granulate according to item 1, wherein said stabilizer is sucrose or polyethylene glycol, preferably sucrose.
3. The granulate according to item 1 or 2, which comprises, as the fesoterodine salt, fesoterodine fumarate.
4. The granulate according to items 1 to 3, free of xylitol, sorbitol, polydextrose, isomalt and dextrose.
5. The granulate according to items 1 to 4, wherein the ratio of fesoterodine or a salt or a solvate thereof, preferably of fesoterodine fumarate, to the stabilizer is in the range of 1:1 to 1:20.
6. A pharmaceutical composition comprising the granulate as defined in any one of items 1 to 5.
7. The pharmaceutical composition according to item 6, which per total dry weight of the composition contains 1-50% of the stabilizer selected from a group consisting of sucrose, polyethylene glycol, cyclodextrin, maltodextrin and combinations thereof, preferably 10-45%
8. The pharmaceutical composition according to item 6 or 7, comprising
   (i) 0.5-10% fesoterodine or a salt or a solvate thereof,
   (ii) 1-50% stabilizer selected from a group consisting of sucrose, polyethylene glycol, cyclodextrin, maltodextrin and combinations thereof,
   (iii) at least one further pharmaceutically acceptable excipient.
9. The pharmaceutical composition according to any of items 6 to 8, comprising
   (i) 1-5% fesoterodine fumarate,
   (ii) 10-45% stabilizer selected from a group consisting of sucrose, polyethylene glycol, cyclodextrin, maltodextrin and combinations thereof
   (iii) 5-65% filler, selected from a group consisting of microcrystalline cellulose, lactose monohydrate, corn starch, calcium phosphate, calcium hydrogenphosphate, composed fillers of microcrystalline cellulose and lactose monohydrate, composed fillers of powdered cellulose and lactose monohydrate and combinations thereof,
   (iv) 0.5-5% glidants, preferably talc,
   (v) 0.5-5% lubricants, preferably glyceryl behenate.
10. The pharmaceutical composition according to any of items 6 to 9 comprising
   (i) 1-5% fesoterodine fumarate,
   (ii) 10-45% stabilizer selected from a group consisting of sucrose, polyethylene glycol, cyclodextrin, maltodextrin and combinations thereof,
   (iii) 5-65% filler, selected from a group consisting of microcrystalline cellulose, lactose monohydrate, corn starch, calcium phosphate, calcium hydrogenphosphate, composed fillers of microcrystalline cellulose and lactose monohydrate, composed fillers of powdered cellulose and lactose monohydrate and combinations thereof,
   (iv) 0.5-5% glidants, selected from a group consisting of talc and colloidal silicon dioxide,
   (v) 0.5-5% lubricants, selected from a group consisting of glyceryl behenate, sodium stearyl fumarate, magnesium stearate, calcium stearat and stearic acid.
11. The pharmaceutical composition according to any of items 6 to 10 comprising
   (i) 1-5% fesoterodine fumarate,
   (ii) 10-45% stabilizer selected from a group consisting of sucrose, polyethylene glycol, cyclodextrin, maltodextrin and combinations thereof,
   (iii) 5-65% filler, selected from a group consisting of microcrystalline cellulose, lactose monohydrate, corn starch, calcium phosphate, calcium hydrogenphosphate, composed fillers of microcrystalline cellulose and lactose monohydrate, composed fillers of powdered cellulose and lactose monohydrate and combinations thereof,
   (iv) 0.5-5% talc,
   (v) 0.5-5% glyceryl behenate.
12. The pharmaceutical composition according to any of items 6 to 11, further comprising a polymer as further excipient.
13. The pharmaceutical composition according to item 12, wherein the polymer is a controlled release polymer, preferably the controlled release polymer is selected from the group consisting of hydroxypropyl methylcellulose, methylcellulose, ethylcellulose, polyethyleneoxide, carrageenan, agar, alginic acid and a mixtures thereof.
14. The pharmaceutical composition according to item 13, wherein the controlled release polymer is hydroxypropyl methylcellulose or a combination of at least two viscosity grades of hydroxypropyl methylcellulose.
15. The pharmaceutical composition according to any one of items 12 to 14, wherein the pharmaceutical composition comprises 15-65% polymer, preferably of the controlled release polymer.
16. The pharmaceutical composition according to any of items 12 to 15, comprising
   (i) 2-5% fesoterodine fumarate,
   (ii) 30-45% sucrose
   (iii) 20-60% microcrystalline cellulose, or composed filler of microcrystalline cellulose and lactose monohydrate
   (iv) 40-50% a mixture of two grades of hydroxypropyl methylcellulose,
   (v) 2-5% talc,
   (vi) 2-5% glyceryl behenate.
17. The pharmaceutical composition according to any of items 12 to 16, comprising
   (i) 2-5% fesoterodine fumarate,
   (ii) 30-45% polyethylene glycol,
   (iii) 20-60% composed filler of microcrystalline cellulose and lactose monohydrate,
   (iv) 40-50% mixture of two grades of hydroxypropyl methylcellulose,
   (v) 2-5% talc,
   (vi) 2-5% glyceryl behenate.
18. The pharmaceutical composition according to any of items 6 to 17, wherein the composition forms tablet cores, respectively coated by a coating, preferably the coating comprises a polymer excipient selected from the group consisting of polyvinyl alcohol, hypromellose, hydroxypropyl cellulose, hydroxyethylcellulose and polymethacrylates.
19. The pharmaceutical composition according to any of items 6 to 18, which when subjected to stability test by exposure to temperature of 60°C and a relative humidity (r.h.) of 21% in open dish for two weeks, the content of hydrolyzed product 5-hydroxymethyltolderodine (5-HMT) measured by HPLC is below 4 wt.-%, preferably 3.0 wt.-% or below, more preferably 2.5 wt.-% or below.
20. A process for preparing the pharmaceutical composition according to any of the items 6 to 19, wherein the process comprises
   (i) granulating fesoterodine or a salt or a solvate thereof with stabilizer selected from a group consisting of sucrose, polyethylene glycol, cyclodextrin, maltodextrin and combinations thereof, and optionally with a filler selected from a group consisting of microcrystalline cellulose, lactose monohydrate, corn starch, calcium phosphate, calcium hydrogenphosphate, composed fillers of microcrystalline cellulose and lactose monohydrate, composed fillers of powdered cellulose and lactose monohydrate and combinations thereof, in the presence of liquid,
   (ii) drying the granulate,
   (iii) optionally screening the granulate,
   (iv) mixing the granulate with at least one other excipient,
   (v) compressing the mixture,
   (vi) optionally applying a coating.
21. The process for preparing the pharmaceutical composition according to item 20, wherein the process comprises
   (i) granulation of fesoterodine or a salt or a solvate thereof with stabilizer selected from a group consisting of sucrose, polyethylene glycol, cyclodextrin, maltodextrin and combinations thereof, and optionally with microcrystalline cellulose in the presence of water,
   (ii) drying the granulate,
   (iii) optionally screening the granulate,
   (iv) mixing the granulate with hydroxypropyl methylcellulose or a mixture of two grades of hydroxypropyl methylcellulose.
   (v) optionally adding talc and glyceryl behenate,
   (vi) compressing the mixture,
   (vii) optionally applying a coating.
22. Use of a substance selected from the group consisting of sucrose, polyethylene glycol, cyclodextrin, maltodextrin and combinations thereof for stabilization of fesoterodine or a salt or a solvate thereof in pharmaceutical composition.
23. The granulate according to any of the items 1 to 5, or the pharmaceutical formulation according to any of the items 6 to 19 for use as a medicament, preferably for use in a treatment of urinary incontinence.

### Description of further advantages and preferred embodiments of the invention

The present invention is now described in more detail by preferred embodiments and examples, which are however presented for illustrative purpose only.

The object of the present invention was to provide an improved pharmaceutical composition with a chemically stable fesoterodine , or a salt or a solvate thereof, as well as a more robust, economical and acceptable production process thereof. In one aspect the present invention provides a granulate comprising fesoterodine, or a salt or a solvate thereof, and a stabilizer selected from a group consisting of sucrose, polyethylene glycol, cyclodextrin, maltodextrin and combinations thereof. In a further aspect said granulate is comprised in a pharmaceutical composition, optionally with further pharmaceutically acceptable excipient(s), the excipient(s) independently from each other being present in the pharmaceutical composition either in the granulate, or in the external part surrounding the granulate, or both.

It was surprisingly found that pharmaceutical composition comprising fesoterodine , or a salt or a solvate thereof, and a stabilizer selected from a group consisting of sucrose, polyethylene glycol, cyclodextrin, maltodextrin and combinations thereof showed an improved stability of fesoterodine. In WO 2007/141298 describes to test stabilizers selected from the group of sugars, sugar alcohols and polyols. WO 2007/141298 teaches that substances selected from sugar alcohols xylitol, sorbitol and isomalt, and the monosaccharide dextrose stabilize fesoterodine in a pharmaceutical composition, however, other substances such as the disaccharide lactose may even have destabilizing effect on fesoterodine and contribute to the degradation of fesoterodine in the pharmaceutical composition. Unexpectedly, it was found that sucrose, a disaccharide showed improved stability of fesoterodine in the common granulate and correspondingly in the pharmaceutical composition. Similarly, an unexpected result of fesoterodine stability in the granulate and correspondingly in the pharmaceutical composition was observed using oligosaccharides, such as cyclodextrin and polysaccharides, such as maltodextrin. Surprisingly, a stabilizing effect was also observed when adding polyethylene glycol to the common granulate and correspondingly in the pharmaceutical composition respectively comprising fesoterodine or a salt or a solvate thereof.

Therefore, a general concept of the present invention is the choice of at least one of substances selected from disaccharides (such as sucrose), oligosaccharides (such as cyclodextrin), polysaccharides (such as maltodextrin) and polyethylene glycols, which substance(s) alone or in combination excerts a stabilizing effect on fesoterodine or a salt or a solvate thereof, preferably a better stabilizing effect than described in WO 2007/141298, wherein the aforementioned substance(s) is (are) added together fesoterodine or a salt or a solvate thereof in a common granulate formulation, which granulate may in turn be comprised in a pharmaceutical composition.

The term "stabilizer" used herein means a pharmaceutically acceptable excipient, that inhibits, prevents, slows down, or reduces the degradation of fesoterodine. The stabilizers may be present in the form of a single compound or in the form of a mixture of compounds.

In a preferred embodiment of the present invention the stabilizer is selected from sucrose and polyethylene glycol. In the most preferred embodiment the stabilizer is sucrose.

In a further aspect the present invention provides a granulate, and pharmaceutical composition comprising said granulate, which granulate or composition comprise 1-50% of stabilizer selected from a group consisting of sucrose, polyethylene glycol, cyclodextrin, maltodextrin and combinations thereof, preferably 10-45%. In a preferred embodiment the fesoterodine , or a salt or a solvate thereof, to stabilizer ratio is 1:1 to 1:20.

In a further aspect the present invention related to a pharmaceutical composition further comprises
about 0.5-10% fesoterodine , or a salt or a solvate thereof,
about 1-50% stabilizer selected from a group consisting of sucrose, polyethylene glycol, cyclodextrin, maltodextrin and combinations thereof,
at least one pharmaceutically acceptable excipient.

The term "about" generally means within 10%, preferably 5% and more preferably within 1% of a given value or range. Alternatively, the term "about" means within an acceptable standard error of the mean, when considered by one of the ordinary skill in the art.

In a preferred embodiment the pharmaceutically acceptable excipient comprises fillers, glidants and lubricants.

The present invention further provides a set of samples of pharmaceutical composition further comprising
about 1-5% fesoterodine , or a salt or a solvate thereof,,
about 10-45% stabilizer selected from a group consisting of sucrose, polyethylene glycol, cyclodextrin, maltodextrin and combinations thereof
about 5-65% filler
about 0.5-5% glidants, and
about 0.5-5% lubricants.

The pharmaceutical compositions described herein can further contain fillers such as microcrystalline cellulose, powdered cellulose, compressible sugar, starch (e.g., corn starch or potato starch), pregelatinized starch, fructose, mannitol, dextranes, other sugars such as, siliconized microcrystalline cellulose, calcium hydrogen phosphate, calcium hydrogen phosphate dihydrate, tricalciumphosphate, calcium lactate or mixtures thereof. The fillers may be present in the form of a single compound or in the form of a mixture of compounds or co-processed compounds.

Preferably, the excipients include at least one filler selected a group of lactose monohydrate, microcrystalline cellulose, a spray-dried compound of lactose monohydrate and microcrystalline cellulose or calcium hydrogenphosphate and composed fillers of microcrystalline cellulose and lactose monohydrate, composed fillers of powdered cellulose and lactose monohydrate and combinations thereof. Preferably, a mixture of lactose and microcrystalline cellulose in a ratio of about 1:1 to about 4:1 [w/w] is used as the filler. A particularly preferred excipient is MICROCELAC® 100, which is a co-processed mixture of lactose monohydrate and microcrystalline cellulose in a ratio of 3:1. Both the filling properties of lactose and the binding capacity of microcrystalline cellulose are synergistically co-processed to one excipient providing improved flow properties and better tabletting performance to the composition.

The compositions described herein may also comprise binders, such as cellulose derivatives (e.g., hypromellose, hydroxypropylcellulose, methylcellulose and sodium carboxymethylcellulose), polyvinylpyrrolidone, gelatin, lactose, sucrose, polyethylene glycol, polymethacrylates, hydroxypropylcellulose, pregelatinized starch and sodium alginate. The term "binder" as used herein is defined as an agent able to bind particles which cannot be bound only by a compression force. The binder may be present in the form of a single compound or in the form of a mixture of compounds.

The compositions described herein may also comprise glidants, such as starches, colloidal silicon dioxide and talc. The term "glidants" as used herein is defined as an agent improving the flow of the powder and thus the filling of the compression chamber of the tablet press. The gliding agent may be present in the pharmaceutical composition in the form of a single compound or in the form of a mixture of compounds. In a preferred embodiment glidant is talc.

The compositions described herein may also comprise lubricants. The term "lubricant" as used herein is defined as an agent able to decrease adhesion of a powder to punches and friction between particles. The lubricant may be present in the pharmaceutical composition in the form of a single compound or in the form of a mixture of compounds. Various suitable lubricants include but are not limited to stearic acid, talc, hydrogenated vegetable oil (*e.g*. hydrogenated castor oil), sodium lauryl sulphate, glyceryl behenate, polyethylene glycol, magnesium stearate, calcium stearate and sodium stearyl fumarate. In a preferred embodiment lubricant is selected from the group consisting of hydrogenated castor oil, polyethylene glycol, glyceryl behenate, magnesium stearate and sodium stearyl fumarate, more preferably lubricant is glyceryl behenate, sodium stearyl fumarate, magnesium stearate, calcium stearate or stearic acid. Most preferably the lubricant is glyceryl behenate.

The present invention further provides a set of samples of pharmaceutical composition according to the previous aspects, wherein the composition comprises
about 1-5% fesoterodine , or a salt or a solvate thereof,
about 10-45% stabilizer selected from a group consisting of sucrose, polyethylene glycol, cyclodextrin, maltodextrin and combinations thereof ,
about 5-65% filler, selected from a group consisting of microcrystalline cellulose, lactose monohydrate, corn starch, calcium phosphate, calcium hydrogenphosphate, composed fillers of microcrystalline cellulose and lactose monohydrate, composed fillers of powdered cellulose and lactose monohydrate and combinations thereof,
about 0.5-5% glidants, selected from a group consisting of talc and colloidal silicon dioxide about 0.5-5% lubricants, selected from a group consisting of glyceryl behenate, sodium stearyl fumarate, magnesium stearate, calcium stearat and stearic acid.

In a preferred embodiment the pharmaceutical composition according to the present invention comprises
about 1-5% fesoterodine , or a salt or a solvate thereof,
about 10-45% stabilizer selected from a group consisting of sucrose, polyethylene glycol, cyclodextrin, maltodextrin and combinations thereof,
about 5-65% selected from a group consisting of microcrystalline cellulose, lactose monohydrate, corn starch, calcium phosphate, calcium hydrogenphosphate, composed fillers of microcrystalline cellulose and lactose monohydrate, composed fillers of powdered cellulose and lactose monohydrate and combinations thereof,,
about 0.5-5% talc,
about 0.5-5% glyceryl behenate.

In a further aspect the present invention provides a pharmaceutical composition further comprises a polymer excipient.

According to one embodiment, the polymer may be a fast dissolving polymer. Suitable fast dissolving polymers include, without being limited thereto, polyethylene glycol 2000 - 6000, polyvinylpyrrolidone, polyvinyl alcohol-polyethylene glycol graft copolymer, polyoxyethylene copolymers, polyoxypropylene copolymers, polyethyleneoxide, hydroxypropyl cellulose, hydroxyethyl cellulose, hydroxyalkyl alkali metal carboxyalkylcellulose derivatives, hydroxyethyl carboxymethyl cellulose, hydroxymethyl carboxyethyl cellulose, hydroxymethyl carboxymethyl cellulose, hydroxypropyl carboxymethyl cellulose, hydroxypropyl carboxyethyl cellulose, hydroxypropyl carboxyethyl cellulose, hydroxypropyl carboxypropyl cellulose, hydroxybutyl carboxymethyl cellulose; or a mixture of any of these compounds.

According to another, preferred embodiment, the polymer is a controlled release polymer. Suitable controlled release polymers include, without being limited thereto, polyethyleneoxide, carrageenan, agar, alginic acid, polyvinylpyrrolidone, polymethacrylate, polyvinylacetate, dextranes, cellulose ethers and esters like methylcellulose, ethylcellulose, methylethylcellulose, hydroxyethylcellulose, hydroxypropylmethylcellulose, hydroxypropylcellulose, or carboxymethylcellulose, or a mixtures thereof. Preferred is hydroxypropylmethylcellulose. In a preferred embodiment the pharmaceutical composition comprises controlled release polymers, selected from a group consisting of at least two viscosity grades of hydroxypropyl methylcellulose.

The particularly preferred controlled release polymers are selected from a group of hydroxypropyl methylcellulose (HPMC), methylcellulose and mixture thereof. HPMC is preferably present in an amount that allows for the formation of a gel matrix from which the active ingredient is gradually released. Particularly preferred brands are METHOCEL® K100M having a nominal viscosity of about 100,000 mPas and METHOCEL® K4M having a nominal viscosity of about 4,000 mPas. The weight ratios of METHOCEL® K100M and K4M used in the compositions described herein can be in the range of about 20:1 to about 1: 20, and are preferably in the range of about 10:1, and are even more preferably in the range of about 5:1.

In a more preferred embodiment the pharmaceutical composition comprises 15-65% controlled release polymers. In the most preferred embodiment the pharmaceutical composition comprises 40-50% controlled release polymers.

In another preferred embodiment, the pharmaceutical composition comprises
about 2-5% fesoterodine fumarate,
about 30-45% sucrose
about 20-60% microcrystalline cellulose, or composed filler of microcrystalline cellulose and lactose monohydrate
about 40-50% a mixture of two grades of hydroxypropyl methylcellulose,
about 2-5% talc,
about 2-5% glyceryl behenate.

In another preferred embodiment, the pharmaceutical composition comprises
about 2-5% fesoterodine fumarate,
about 30-45% polyethylene glycol,
about 20-60% composed filler of microcrystalline cellulose and lactose monohydrate,
about 40-50% mixture of two grades of hydroxypropyl methylcellulose,
about 2-5% talc,
about 2-5% glyceryl behenate.

In the above specifications relating to aspects and embodiments of pharmaceutical compositions, generally the fesoterodine compound and the stabilizer, optionally a part or all of filler, are provided in the granulate, and optionally another part or all of filler and glidant and/or lubricant are provided as exteragranular exipients of the respective pharmaceutical compositions. Since the stabilizer selected from sucrose, polyethylene glycol, cyclodextrin and maltodextrin is present in the granulate together with fesoterodine, or a salt or a solvate thereof, the same stabilizer compound may, if desired, be added also the external excipient to be mixed with the granulate, however in terms of stability this is not needed and therefore said stabilizer compound can be omitted in such external excipients.

In another aspect, the present invention further provides a pharmaceutical composition further comprising a coating containing a polymer excipient selected from a group consisting of polyvinyl alcohol, hypromellose, hydroxypropyl cellulose, hydroxyethylcellulose, polymethacrylates.

The term "coating" as used herein refers to a layer which completely covers an object and is applied by film coating. The coating can be selected from the group of ready to form dispersion such as OPADRY. The coating dispersion comprises hydrophilic film forming polymer (such as for example low viscosity HPMC, HPC, PVA (polyvinylalcohol) and the like), plastificators (e.g. PEG), colorants and may optionally include other excipients such as antitacking agents.

Any method for film coating, known in the field of the pharmaceutical technology, may be used.

A pharmaceutical compositions according to the present invention is preferably in solid form, including tablets, capsules, caplets, lozenges and sachets. Tablets may be suitably coated (film coated tablets, pills). Capsule formulations may cover both soft and hard capsules. A pharmaceutical compositions according to the present invention is preferably in the form of tablet, more preferably coated tablet with appropriate film coating material.

In another aspect, the present invention further provides a pharmaceutical composition, which when subjected to stability test by exposure to temperature of 60°C and a relative humidity (r.h.) of 21% in open dish for two weeks, the content of hydrolyzed product 5-hydroxymethyltolderodine (5-HMT) measured by HPLC is below 4 wt.-%, preferably 3.0 wt.-% or below, more preferably 2.5 wt.-% or below.

In another aspect, the present invention further provides a pharmaceutical composition for use in the preparation of a medicament for the treatment of urinary incontinence.

In another aspect, the present invention further provides a process for preparing the pharmaceutical composition comprising fesoterodine, or a salt or a solvate thereof, and stabilizer. The granulate can be made by dry granulation, which includes the possibility of using up to maximally 10 wt.% liquid such as water, alcohol (such as ethanol) or other liquid in a moisture-activated dry granulation, or by wet granulation.

Surprisingly, it has been found that using wet granulation process, wherein granulating API and stabilizer with the addition of standard excipients for wet granulation showed an improved stability of fesoterodine in the granulate and in the pharmaceutical composition including the granulate. Prior art teaches the wet granulation with only API and stabilizer as the preferred process for preparation of pharmaceutical composition comprising fesoterodine fumarate. Wet granulation of only API and stabilizer without the addition of standard excipients for wet granulation such as lactose and microcrystalline cellulose is more complicated from technological point of view than when the granulate contains also lactose an microcrystalline cellulose; these two excipients contribute to optimal consistence of wet and dried granulate, its non-stickiness and less sensitivity to the amount of water added. Additionally, it has been found to be advantageous when the granulate comprises more excipients and represents the greater portion of the tablets, due to easier manufacturing equipment utilization.

In a preferred embodiment the process for preparing the pharmaceutical composition comprises
granulation of fesoterodine or a salt or a solvate thereof with stabilizer selected from a group consisting of sucrose, polyethylene glycol, cyclodextrin, maltodextrin and combinations thereof and optionally a filler selected from a group consisting of microcrystalline cellulose, lactose monohydrate, corn starch, calcium phosphate, calcium hydrogenphosphate, composed fillers of microcrystalline cellulose and lactose monohydrate, composed fillers of powdered cellulose and lactose monohydrate and combinations thereof, in the presence of liquid,
drying the granulate,
optionally screening the granulate,
mixing the granulate with at least one other excipient,
compressing the mixture,
optionally applying a coating.

In the most preferred embodiment the process for preparing the pharmaceutical composition further comprises
granulation of fesoterodine or a salt or a solvate thereof with stabilizer selected from a group consisting of sucrose, polyethylene glycol, cyclodextrin, maltodextrin and combinations thereof and optionally microcrystalline cellulose in the presence of water,
drying the granulate,
optionally screening the granulate,
mixing the granulate with hydroxypropyl methylcellulose or a mixture of two grades of hydroxypropyl methylcellulose,
optionally adding talc and glyceryl behenate,
compressing the mixture,
optionally applying a coating.

In another aspect the present invention relates to use of a substance selected from the group consisting of sucrose, polyethylene glycol, cyclodextrin, maltodextrin and combinations thereof for stabilization of fesoterodine or a salt or a solvate thereof in pharmaceutical composition.

Surprisingly, it has been found that substances selected from a group consisting of sucrose, polyethylene glycol, cyclodextrin, maltodextrin and combinations thereof can be used for stabilizing fesoterodine or a salt or a solvate thereof in a pharmaceutical composition. WO 2007/141298 teaches that substances selected from the group of sugar alcohols, such as xylitol, sorbitol, isomalt and monosaccharides, such as dextrose can be used as stabilizers of fesoterodine in a pharmaceutical composition, however, substances selected from disaccharides, such as lactose have destabilizing effect on fesoterodine and contribute to the degradation of fesoterodine in the pharmaceutical composition. Unexpectedly, it has been found that sucrose, a disaccharide may be used for stabilizing fesoterodine in the pharmaceutical composition. Similarly, an unexpected result of the use of oligosaccharides, such as cyclodextrin and polysaccharides, such as maltodextrin for stabilizing fesoterodine in the pharmaceutical composition was observed. Surprisingly, polyethylene glycol can be also used as a stabilizer in pharmaceutical composition comprising fesoterodine or a salt or a solvate thereof.

### Examples

### Example 1

| Component | Amount per tablet (mg) | | Amount per lab. batch (g) |
|---|---|---|---|
| GRANULATE | | | |
| Fesoterodine fumarate | 8.000 | 2.5% | 12.500 |
| Sucrose | 72.000 | 22.5% | 112.500 |
| Total | 80.000 | 25.0% | 125.000 |
| TABLETS | | | |
| Granulate | 80.000 | 25.0% | 100.000 |
| Microcrystalline cellulose | 20.000 | 6.3% | 25.000 |
| Lactose monohydrate | 58.000 | 18.1% | 72.500 |
| Hypromellose Methocel K100M | 120.000 | 37.5% | 150.000 |
| Hypromellose Methocel K4M | 24.000 | 7.5% | 30.000 |
| Gliceryl Behenate | 10.000 | 3.1% | 12.500 |
| Talc | 8.000 | 2.5% | 10.000 |
| Total | 320.000 | 100.0% | 400.000 |

Fesoterodine fumarate and sucrose were granulated with demineralized water in high shear mixer. Granulate was dried in vacuum chamber drier to water content (los-on-drying - LOD) less than 0.5%. Dried granulate was pushed through 0.75 mm screen on oscillating bar mill. Granulate was mixed with microcrystalline cellulose, lactose, Methocel K4M and Methocel K100M in cubic bin blender for 5 min. Then talc was added to the blend in blender, and mixed for 2 min. Then glyceryl behenate was added to the blend in blender and mixed for 2 min, so that final tabletting blend was obtained.

Final blend was compressed into oval biconvex tablets by rotary tabletting machine.

### Example 2

| Component | Amount per tablet (mg) | | Amount per lab. batch (g) |
|---|---|---|---|
| GRANULATE | | | |
| Fesoterodine fumarate | 8.000 | 2.5% | 11.111 |
| Sucrose | 72.000 | 22.5% | 100.000 |
| Microcrystalline cellulose | 20.000 | 6.3% | 27.778 |
| Lactose monohydrate | 58.000 | 18.1% | 80.556 |
| Total | 158.000 | 49.4% | 219.444 |
| TABLETS | | | |
| Granulate | 158.000 | 49.4% | 197.500 |
| Hypromellose Methocel K100M | 120.000 | 37.5% | 150.000 |
| Hypromellose Methocel K4M | 24.000 | 7.5% | 30.000 |
| Gliceryl Behenate | 10.000 | 3.1% | 12.500 |
| Talc | 8.000 | 2.5% | 10.000 |
| Total | 320.000 | 100.0% | 400.000 |

Fesoterodine fumarate, sucrose, microcrystalline cellulose and lactose were granulated with demineralized water in high shear mixer. Granulate was dried in vacuum chamber drier to water content (los-on-drying - LOD) less than 0.5%. Dried granulate was pushed through 0.75 mm screen on oscillating bar mill. Granulate was mixed with Methocel K4M and Methocel K100M in cubic bin blender for 5 min. Then talc was added to the blend in blender, and mixed for 2 min. Then glyceryl behenate was added to the blend in blender and mixed for 2 min, so that final tabletting blend was obtained. Final blend was compressed into oval biconvex tablets by rotary tabletting machine.

### Example 3

| Component | Amount per tablet (mg) | | Amount per lab. batch (g) |
|---|---|---|---|
| GRANULATE | | | |
| Fesoterodine fumarate | 8.000 | 2.5% | 11.111 |
| Sucrose | 130.000 | 40.6% | 180.556 |
| Microcrystalline cellulose | 20.000 | 6.3% | 27.778 |
| Total | 158.000 | 49.4% | 219.444 |
| TABLETS | | | |
| Granulate | 158.000 | 49.4% | 197.500 |
| Hypromellose Methocel K100M | 120.000 | 37.5% | 150.000 |
| Hypromellose Methocel K4M | 24.000 | 7.5% | 30.000 |
| Gliceryl Behenate | 10.000 | 3.1% | 12.500 |
| Talc | 8.000 | 2.5% | 10.000 |
| Total | 320.000 | 100.0% | 400.000 |

Fesoterodine fumarate, sucrose, microcrystalline cellulose and lactose were granulated with demineralized water in high shear mixer. Granulate was dried in vacuum chamber drier to water content (los-on-drying - LOD) less than 0.5%. Dried granulate was pushed through 0.75 mm screen on oscillating bar mill. Granulate was mixed with Methocel K4M and Methocel K100M in cubic bin blender for 5 min. Then talc was added to the blend in blender, and mixed for 2 min. Then glyceryl behenate was added to the blend in blender and mixed for 2 min, so that final tabletting blend was obtained. Final blend was compressed into oval biconvex tablets by rotary tabletting machine.

### Example 4

| Component | Amount per tablet (mg) | | Amount per lab. batch (g) |
|---|---|---|---|
| GRANULATE | | | |
| Fesoterodine fumarate | 8.000 | 2.5% | 11.111 |
| Sucrose | 100.000 | 31.3% | 138.889 |
| Microcrystalline cellulose | 50.000 | 15.6% | 69.444 |
| Total | 158.000 | 49.4% | 219.444 |
| TABLETS | | | |
| Granulate | 158.000 | 49.4% | 197.500 |
| Hypromellose Methocel K100M | 120.000 | 37.5% | 150.000 |
| Hypromellose Methocel K4M | 24.000 | 7.5% | 30.000 |
| Gliceryl Behenate | 10.000 | 3.1% | 12.500 |
| Talc | 8.000 | 2.5% | 10.000 |
| Total | 320.000 | 100.0% | 400.000 |

Fesoterodine fumarate, sucrose, microcrystalline cellulose and lactose were granulated with demineralized water in high shear mixer. Granulate was dried in vacuum chamber drier to water content (los-on-drying - LOD) less than 0.5%. Dried granulate was pushed through 0.75 mm screen on oscillating bar mill. Granulate was mixed with Methocel K4M and Methocel K100M in cubic bin blender for 5 min. Then talc was added to the blend in blender, and mixed for 2 min. Then glyceryl behenate was added to the blend in blender and mixed for 2 min, so that final tabletting blend was obtained. Final blend was compressed into oval biconvex tablets by rotary tabletting machine.

### Example 5

| Component | Amount per tablet (mg) | | Amount per lab. batch (g) |
|---|---|---|---|
| GRANULATE | | | |
| Fesoterodine fumarate | 8.000 | 2.5% | 0.750 |
| Polyethylene glycol 6000 | 72.000 | 22.5% | 6.750 |
| Total | 80.000 | 25.0% | 7.500 |
| TABLETS | | | |
| Granulate | 80.000 | 25.0% | 7.500 |
| Microcrystalline cellulose | 20.000 | 6.3% | 1.875 |
| Lactose monohydrate | 58.000 | 18.1% | 5.438 |
| Hypromellose Methocel K100M | 120.000 | 37.5% | 11.250 |
| Hypromellose Methocel K4M | 24.000 | 7.5% | 2.250 |
| Gliceryl Behenate | 10.000 | 3.1% | 0.938 |
| Talc | 8.000 | 2.5% | 0.750 |
| Total | 320.000 | 100.0% | 30.000 |

Fesoterodine fumarate and polyethylene glycol were granulated with demineralized water manually in mortar with pestle. Granulate was dried in vacuum chamber drier to water activity less than 20%. Dried granulate was pushed through 0.75 mm screen. Granulate was mixed with microcrystalline cellulose, lactose, Methocel K4M and Methocel K100M manually in polyethylene (PE) bag for 3 min. Then talc was added to the blend in PE bag, and mixed for 1 min. Then glyceryl behenate was added to the blend in PE bag and mixed for 1 min, so that final tabletting blend was obtained. Final blend was compressed into oval biconvex tablets by eccentric tabletting machine.

### Example 6

| Component | Amount per tablet (mg) | | Amount per lab. batch (g) |
|---|---|---|---|
| GRANULATE | | | |
| Fesoterodine fumarate | 8.000 | 2.5% | 0.750 |
| Polyethylene glycol 6000 | 72.000 | 22.5% | 6.750 |
| Microcrystalline cellulose | 20.000 | 6.3% | 1.875 |
| Lactose monohydrate | 58.000 | 18.1% | 5.438 |
| Total | 158.000 | 49.4% | 14.813 |

| TABLETS | | | |
|---|---|---|---|
| Granulate | 158.000 | 49.4% | 14.813 |
| Hypromellose Methocel K100M | 120.000 | 37.5% | 11.250 |
| Hypromellose Methocel K4M | 24.000 | 7.5% | 2.250 |
| Gliceryl Behenate | 10.000 | 3.1% | 0.938 |
| Talc | 8.000 | 2.5% | 0.750 |
| Total | 320.000 | 100.0% | 30.000 |

Fesoterodine fumarate, polyethylene glycol microcrystalline cellulose and lactose, were granulated with demineralized water manually in mortar with pestle. Granulate was dried in vacuum chamber drier to water activity less than 20%. Dried granulate was pushed through 0.75 mm screen. Granulate was mixed with Methocel K4M and Methocel K100M manually in polyethylene (PE) bag for 3 min. Then talc was added to the blend in PE bag, and mixed for 1 min. Then glyceryl behenate was added to the blend in PE bag and mixed for 1 min, so that final tabletting blend was obtained. Final blend was compressed into oval biconvex tablets by eccentric tabletting machine.

### Example 7

| Component | Amount per tablet (mg) | | Amount per lab. batch (g) |
|---|---|---|---|
| GRANULATE | | | |
| Fesoterodine fumarate | 8.000 | 2.5% | 0.750 |
| Maltodextrin | 72.000 | 22.5% | 6.750 |
| Total | 80.000 | 25.0% | 7.500 |
| TABLETS | | | |
| Granulate | 80.000 | 25.0% | 7.500 |
| Microcrystalline cellulose | 20.000 | 6.3% | 1.875 |
| Lactose monohydrate | 58.000 | 18.1% | 5.438 |
| Hypromellose Methocel K100M | 120.000 | 37.5% | 11.250 |
| Hypromellose Methocel K4M | 24.000 | 7.5% | 2.250 |
| Gliceryl Behenate | 10.000 | 3.1% | 0.938 |
| Talc | 8.000 | 2.5% | 0.750 |
| Total | 320.000 | 100.0% | 30.000 |

Fesoterodine fumarate and maltodextrin were granulated with demineralized water manually in mortar with pestle. Granulate was dried in vacuum chamber drier to water activity less than 20%. Dried granulate was pushed through 0.5 mm screen. Granulate was mixed with mcrocrystalline cellulose, lactose, Methocel K4M and Methocel K100M manually in polyethylene (PE) bag for 3 min. Then talc was added to the blend in PE bag, and mixed for 1 min. Then glyceryl behenate was added to the blend in PE bag and mixed for 1 min, so that final tabletting blend was obtained. Final blend was compressed into oval biconvex tablets by eccentric tabletting machine.

### Example 8

| Component | Amount per tablet (mg) | | Amount per lab. batch (g) |
|---|---|---|---|
| GRANULATE | | | |
| Fesoterodine fumarate | 8.000 | 2.5% | 0.750 |
| Maltodextrin | 72.000 | 22.5% | 6.750 |
| Microcrystalline cellulose | 20.000 | 6.3% | 1.875 |
| Lactose monohydrate | 58.000 | 18.1% | 5.438 |
| Total | 158.000 | 49.4% | 14.813 |
| TABLETS | | | |
| Granulate | 158.000 | 49.4% | 14.813 |
| Hypromellose Methocel K100M | 120.000 | 37.5% | 11.250 |
| Hypromellose Methocel K4M | 24.000 | 7.5% | 2.250 |
| Gliceryl Behenate | 10.000 | 3.1% | 0.938 |
| Talc | 8.000 | 2.5% | 0.750 |
| Total | 320.000 | 100.0% | 30.000 |

Fesoterodine fumarate, maltodextrin, microcrystalline cellulose and lactose were granulated with demineralized water manually in mortar with pestle. Granulate was dried in vacuum chamber drier to water activity less than 20%. Dried granulate was pushed through 0.75 mm screen. Granulate was mixed with Methocel K4M and Methocel K100M manually in polyethylene (PE) bag for 3 min. Then talc was added to the blend in PE bag, and mixed for 1 min. Then glyceryl behenate is added to the blend in PE bag and mixed for 1 min, so that final tabletting blend was obtained. Final blend was compressed into oval biconvex tablets by eccentric tabletting machine.

### Example 9

| Component | Amount per tablet (mg) | | Amount per lab. batch (g) |
|---|---|---|---|
| GRANULATE | | | |
| Fesoterodine fumarate | 8.000 | 2.5% | 0.750 |
| Hydroxypropyl- β-cyclodextrin | 72.000 | 22.5% | 6.750 |
| Total | 80.000 | 25.0% | 7.500 |
| TABLETS | | | |
| Granulate | 80.000 | 25.0% | 7.500 |
| Microcrystalline cellulose | 20.000 | 6.3% | 1.875 |
| Lactose monohydrate | 58.000 | 18.1% | 5.438 |
| Hypromellose Methocel K100M | 120.000 | 37.5% | 11.250 |
| Hypromellose Methocel K4M | 24.000 | 7.5% | 2.250 |
| Gliceryl Behenate | 10.000 | 3.1% | 0.938 |
| Talc | 8.000 | 2.5% | 0.750 |
| Total | 320.000 | 100.0% | 30.000 |

Fesoterodine fumarate and HP- β-cyclodextrin were granulated with demineralized water manually in mortar with pestle. Granulate was dried in vacuum chamber drier to water activity less than 20%. Dried granulate was pushed through 0.75 mm screen. Granulate was mixed with microcrystalline cellulose, lactose, Methocel K4M and Methocel K100M manually in polyethylene (PE) bag for 3 min. Then talc was added to the blend in PE bag, and mixed for 1 min. Then glyceryl behenate was added to the blend in PE bag and mixed for 1 min, so that final tabletting blend was obtained. Final blend was compressed into oval biconvex tablets by eccentric tabletting machine.

### Example 10

| Component | Amount per tablet (mg) | | Amount per lab. batch (g) |
|---|---|---|---|
| GRANULATE | | | |
| Fesoterodine fumarate | 8.000 | 2.5% | 0.750 |
| Hydroxypropyl- β-cyclodextrin | 72.000 | 22.5% | 6.750 |
| Microcrystalline cellulose | 20.000 | 6.3% | 1.875 |
| Lactose monohydrate | 58.000 | 18.1% | 5.438 |
| Total | 158.000 | 49.4% | 14.813 |
| TABLETS | | | |
| Granulate | 158.000 | 49.4% | 14.813 |
| Hypromellose Methocel K100M | 120.000 | 37.5% | 11.250 |
| Hypromellose Methocel K4M | 24.000 | 7.5% | 2.250 |
| Gliceryl Behenate | 10.000 | 3.1% | 0.938 |
| Talc | 8.000 | 2.5% | 0.750 |
| Total | 320.000 | 100.0% | 30.000 |

Fesoterodine fumarate, HP- β-cyclodextrin, microcrystalline cellulose and lactose were granulated with demineralized water manually in mortar with pestle. Granulate was dried in vacuum chamber drier to water activity less than 20%. Dried granulate was pushed through 0.75 mm screen. Granulate was mixed with Methocel K4M and Methocel K100M manually in polyethylene (PE) bag for 3 min. Then talc was added to the blend in PE bag, and mixed for 1 min. Then glyceryl behenate was added to the blend in PE bag and mixed for 1 min, so that final tabletting blend was obtained. Final blend was compressed into oval biconvex tablets by eccentric tabletting machine.

### Comparative example A- prepared according to WO2007/141298

| Component | Amount per tablet (mg) | | Amount per lab. batch (g) |
|---|---|---|---|
| GRANULATE | | | |
| Fesoterodine fumarate | 8.000 | 2.5% | 8.000 |
| Xylitol | 72.000 | 22.5% | 72.000 |
| Total | 80.000 | 25.0% | 80.000 |
| TABLETS | | | |
| Granulate | 80.000 | 25.0% | 40.000 |
| Lactose monohydrate | 58.125 | 18.2% | 29.063 |
| Microcrystalline cellulose | 19.375 | 6.1% | 9.688 |
| Hypromellose Methocel K100M | 120.000 | 37.5% | 60.000 |
| Hypromellose Methocel K4M | 24.000 | 7.5% | 12.000 |
| Gliceryl Behenate | 10.000 | 3.1% | 5.000 |
| Talc | 8.500 | 2.7% | 4.250 |
| Total | 320.000 | 100.0% | 820.000 |

Fesoterodine fumarate and xylitol were granulated with demineralized water manually in mortar with pestle. Granulate was dried in vacuum chamber drier to LOD less than 0.5%. Dried granulate was pushed through 0.8 mm screen. Granulate was mixed with lactose and MCC and passed through screen 0.8 mm. Then Methocel K4M and Methocel K100M were added to the screened mixture and blended in double-cone blender for 5 min. The mixture was then again passed through screen 0.8 mm and blended in double-cone blender for 10 min so that final tabletting blend was obtained. Final blend was compressed into oval biconvex tablets by rotary tabletting machine.

### Comparative example B

| Component | Amount per tablet (mg) | | Amount per lab. batch (g) |
|---|---|---|---|
| GRANULATE | | | |
| Fesoterodine fumarate | 8.000 | 2.5% | 0.750 |
| Microcrystalline cellulose | 70.000 | 21.9% | 6.563 |
| Hypromellose Pharmacoat 606 | 2.000 | 0.6% | 0.188 |
| Total | 80.000 | 25.0% | 7.500 |
| TABLETS | | | |
| Granulate | 80.000 | 25.0% | 7.500 |
| Lactose monohydrate | 78.000 | 24.4% | 7.313 |
| Hypromellose Methocel K100M | 120.000 | 37.5% | 11.250 |
| Hypromellose Methocel K4M | 24.000 | 7.5% | 2.250 |
| Gliceryl Behenate | 10.000 | 3.1% | 0.938 |
| Talc | 8.000 | 2.5% | 0.750 |
| Total | 320.000 | 100.0% | 30.000 |

Fesoterodine fumarate, microcrystalline cellulose and Hypromellose Pharmacoat 606 were granulated with demineralized water manually in mortar with pestle. Granulate was dried in vacuum chamber drier to water activity less than 20%. Dried granulate was pushed through 0,75 mm screen. Granulate was mixed with lactose, Methocel K4M and Methocel K100M manually in polyethylene (PE) bag for 3 min. Then talc was added to the blend in PE bag, and mixed for 1 min. Then glyceryl behenate was added to the blend in PE bag and mixed for 1 min, so that final tabletting blend was obtained. Final blend was compressed into oval biconvex tablets by eccentric tabletting machine.

### Comparative example C

| Component | Amount per tablet (mg) | | Amount per lab. batch (g) |
|---|---|---|---|
| GRANULATE | | | |
| Fesoterodine fumarate | 8.000 | 2.5% | 0.750 |
| Microcrystalline cellulose | 70.000 | 21.9% | 6.563 |
| Polyvinylpyrrolidone | 2.000 | 0.6% | 0.188 |
| Total | 80.000 | 25.0% | 7.500 |
| TABLETS | | | |
| Granulate | 80.000 | 25.0% | 7.500 |
| Lactose monohydrate | 78.000 | 24.4% | 7.313 |
| Hypromellose Methocel K100M | 120.000 | 37.5% | 11.250 |
| Hypromellose Methocel K4M | 24.000 | 7.5% | 2.250 |
| Gliceryl Behenate | 10.000 | 3.1% | 0.938 |
| Talc | 8.000 | 2.5% | 0.750 |
| Total | 320.000 | 100.0% | 30.000 |

Fesoterodine fumarate, microcrystalline cellulose and Polyvinylpyrrolidone were granulated with demineralized water manually in mortar with pestle. Granulate was dried in vacuum chamber drier to water activity less than 20%. Dried granulate was pushed through 0.75 mm screen. Granulate was mixed with lactose, Methocel K4M and Methocel K100M manually in polyethylene (PE) bag for 3 min. Then talc was added to the blend in PE bag and mixed for 1 min. Then glyceryl behenate was added to the blend in PE bag and mixed for 1 min so that final tabletting blend was obtained. Final blend was compressed into oval biconvex tablets by eccentric tabletting machine.

### Comparative example D

| Component | Amount per tablet (mg) | | Amount per lab. batch (g) |
|---|---|---|---|
| GRANULATE | | | |
| Fesoterodine fumarate | 8.000 | 2.5% | 0.750 |
| Calcium hydrogenphosphate | 70.000 | 21.9% | 6.563 |
| Hypromellose Pharmacoat 606 | 2.000 | 0.6% | 0.188 |
| Total | 80.000 | 25.0% | 7.500 |
| TABLETS | | | |
| Granulate | 80.000 | 25.0% | 7.500 |
| Lactose monohydrate | 78.000 | 24.4% | 7.313 |
| Hypromellose Methocel K100M | 120.000 | 37.5% | 11.250 |
| Hypromellose Methocel K4M | 24.000 | 7.5% | 2.250 |
| Gliceryl Behenate | 10.000 | 3.1% | 0.938 |
| Talc | 8.000 | 2.5% | 0.750 |
| Total | 320.000 | 100.0% | 30.000 |

Fesoterodine fumarate, calcium hydrogenphosphate and Hypromellose Pharmacoat 606 were granulated with demineralized water manually in mortar with pestle. Granulate was dried in vacuum chamber drier to water activity less than 20%. Dried granulate was pushed through 0.75 mm screen. Granulate was mixed with lactose, Methocel K4M and Methocel K100M manually in polyethylene (PE) bag for 3 min. Then talc was added to the blend in PE bag, and mixed for 1 min. Then glyceryl behenate was added to the blend in PE bag and mixed for 1 min, so that final tabletting blend was obtained. Final blend was compressed into oval biconvex tablets by eccentric tabletting machine.

### Stress stability tests

A stress stability test was designed to show intrinsic stability of test formulations in comparison to a reference formulation, known from prior art. The test samples (tablets) were exposed to elevated temperature (60°C) and a controlled relative humidity (21%). The appropriate relative humidity was achieved by means of a saturated solution of potassium fluoride, stored in an impermeable cabined inside a thermostatically controlled chamber, se tat 60°C. Tablets were exposed to this stress condition for two weeks with no primary packaging (open dish). After two weeks, samples were analyzed for content of 5-HMT by means of high performance liquid chromatograph (HPLC).

HPLC method was run on an Alliance HPLC instrument (Waters) using BEH Shield RP18 column (50 x 2.1 mm 1.7 um particles). The mobile phase consisted of 0.05% phosphoric acid (A) and acetonitrile (B). The mobile phase was pumped at 0.5 ml/min with a gradient, beginning at 10% mobile phase B, increasing to 90% mobile phase B in 4 minutes, followed by column reequilibration for two minutes. Chromatograms were followed at 220 nm and typical retention times of fesoterodine fumarate and 5-HMT were 2.5 and 1.5 min, respectively. Evaluation is done by the Area% method

### Stability evaluation:

A formulation is preferred, if the amount of 5-HMT, formed after exposure to the indicated stress conditions, is below or equal to that of the comparative example.

| | % 5-HMT (hydrolysis product) | Total degradation products |
|---|---|---|
| Example 1 | 1.8 | 5.6 |
| Example 2 | 2.0 | 7.1 |
| Example 3 | 1.7 | 5.1 |
| Example 4 | 1.8 | 5.8 |
| Example 5 | 0.7 | 1.8 |
| Example 6 | 2.1 | 4.9 |
| Example 7 | 2.9 | 7.8 |
| Example 8 | 4.3 | 15.9 |
| Example 9 | 2.4 | 5.5 |
| Example 10 | 1.6 | 3.3 |
| Comparative example A | 4.0 | 10.8 |
| Comparative example B | 6.6 | 18.4 |
| Comparative example C | 8.2 | 21 |
| Comparative example D | 9.3 | 37 |

## Claims

1. A granulate comprising fesoterodine or a salt or a solvate thereof, and a stabilizer selected from a group consisting of sucrose, polyethylene glycol, cyclodextrin, maltodextrin and combinations thereof.

2. The granulate according to claim 1, wherein said stabilizer is sucrose or polyethylene glycol, preferably sucrose.

3. The granulate according to claim 1 or 2, which comprises, as the fesoterodine salt, fesoterodine fumarate.

4. The granulate according to claims 1 to 3, free of xylitol, sorbitol, polydextrose, isomalt and dextrose.

5. The granulate according to claims 1 to 4, wherein the ratio of fesoterodine or a salt or a solvate thereof, preferably of fesoterodine fumarate, to the stabilizer is in the range of 1:1 to 1:20.

6. A pharmaceutical composition comprising the granulate as defined in any one of claims 1 to 5.

7. The pharmaceutical composition according to claim 6, comprising
(i) 0.5-10% fesoterodine or a salt or a solvate thereof,
(ii) 1-50% stabilizer selected from a group consisting of sucrose, polyethylene glycol, cyclodextrin, maltodextrin and combinations thereof,
(iii) at least one further pharmaceutically acceptable excipient.

8. The pharmaceutical composition according to claim 6 or 7, comprising
(i) 1-5% fesoterodine fumarate,
(ii) 10-45% stabilizer selected from a group consisting of sucrose, polyethylene glycol, cyclodextrin, maltodextrin and combinations thereof
(iii) 5-65% filler, selected from a group consisting of microcrystalline cellulose, lactose monohydrate, corn starch, calcium phosphate, calcium hydrogenphosphate, composed fillers of microcrystalline cellulose and lactose monohydrate, composed fillers of powdered cellulose and lactose monohydrate and combinations thereof,
(iv) 0.5-5% glidants, preferably talc,
(v) 0.5-5% lubricants, preferably glyceryl behenate.

9. The pharmaceutical composition according to any of claims 6 to 8, further comprising a polymer as further excipient, preferably a controlled release polymer, wherein more preferably the controlled release polymer is selected from the group consisting of hydroxypropyl methylcellulose, methylcellulose hydroxyethylcellulose, hydroxypropylcellulose, carboxymethylcellulose, ethylcellulose, polyethyleneoxide, carrageenan, agar, alginic acid, pectin and a mixtures thereof, preferably hydroxypropyl methylcellulose..

10. The pharmaceutical composition according to claim 9, wherein the controlled release polymer is a combination of at least two viscosity grades of hydroxypropyl methylcellulose.

11. The pharmaceutical composition according to claim 9 or 10, comprising
(i) 2-5% fesoterodine fumarate,
(ii) 30-45% sucrose
(iii) 20-60% microcrystalline cellulose, or composed filler of microcrystalline cellulose and lactose monohydrate
(iv) 40-50% a mixture of two grades of hydroxypropyl methylcellulose,
(v) 2-5% talc,
(vi) 2-5% glyceryl behenate.

12. The pharmaceutical composition according to any of claims 6 to 11, wherein the composition forms tablet cores, respectively coated by a coating, preferably the coating comprises a polymer excipient selected from the group consisting of polyvinyl alcohol, hypromellose, hydroxypropyl cellulose, hydroxyethylcellulose and polymethacrylates.

13. The pharmaceutical composition according to any of claims 6 to 12, which when subjected to stability test by exposure to temperature of 60°C and a relative humidity (r.h.) of 21% in open dish for two weeks, the content of hydrolyzed product 5-hydroxymethyltolderodine (5-HMT) measured by HPLC is below 4 wt.-%, preferably 3.0 wt.-% or below, more preferably 2.5 wt.-% or below.

14. A process for preparing the pharmaceutical composition according to any of the claims 6 to 13, wherein the process comprises
(i) granulating fesoterodine or a salt or a solvate thereof with stabilizer selected from a group consisting of sucrose, polyethylene glycol, cyclodextrin, maltodextrin and combinations thereof, and optionally with a filler selected from a group consisting of microcrystalline cellulose, lactose monohydrate, corn starch, calcium phosphate, calcium hydrogenphosphate, composed fillers of microcrystalline cellulose and lactose monohydrate, composed fillers of powdered cellulose and lactose monohydrate and combinations thereof, in the presence of liquid,
(ii) drying the granulate,
(iii) optionally screening the granulate,
(iv) mixing the granulate with at least one other excipient,
(v) compressing the mixture,
(vi) optionally applying a coating.

15. Use of a substance selected from the group consisting of sucrose, polyethylene glycol, cyclodextrin, maltodextrin and combinations thereof for stabilization of fesoterodine or a salt or a solvate thereof in pharmaceutical composition.

16. The granulate according to any of the claims 1 to 5, or the pharmaceutical formulation according to any of the claims 6 to 13 for use as a medicament, preferably for use in a treatment of urinary incontinence.
